# FASCICULE DE BREVET EUROPEEN

(11) **EP 2 352 984 B1**
(45) Date de publication et mention de la délivrance du brevet: **22.05.2019**
(21) Numéro de dépôt: 09801729.6
(22) Date de dépôt: 01.12.2009
(51) Int. Cl.: G01N 15/12, G01N 15/14

(54) **PROCEDE ET DISPOSITIF DE CYTOMETRIE EN FLUX SANS FLUIDE DE GAINAGE**
DURCHFLUSSZYTOMETRIEVERFAHREN UND-VORRICHTUNG OHNE HÜLLFLÜSSIGKEIT
SHEATH-FLUID-FREE FLOW CYTOMETRY METHOD AND DEVICE

(30) Priorité: 02.12.2008 FR 0858200
(43) Date de publication de la demande: 10.08.2011
(73) Titulaire: BIT Group France, 34099 Montpellier (FR)
(72) Inventeur: CHAMPSEIX, Henri, F-34980 Saint Gely du Fesc (FR); MAGNIN, Olivier, F-38530 Chapareillan (FR); WELMANT, Bernard, F-34730 PRADES LE LEZ (FR)
(74) Mandataire: IPAZ
(86) Numéro de dépôt international: PCT/FR2009/052349
(87) Numéro de publication internationale: WO 2010/063936

(56) Documents cités:
- WO-A2-01/69202
- CH-A- 544 305
- FR-A- 2 873 813
- US-A- 3 710 264
- US-A- 4 420 720
- US-A- 4 633 169
- US-A- 4 797 624
- US-A1- 2001 052 763
- US-A1- 2006 160 229
- US-A1- 2008 093 216
- US-B1- 6 614 215
- VON BEHRENS W ET AL: "COMPARISON OF TECHNIQUES IMPROVING THE RESOLUTION OF STANDARD COULTER CELL SIZING SYSTEMS" 1976, JOURNAL OF HISTOCHEMISTRY AND CYTOCHEMISTRY, VOL. 24, NR. 1, PAGE(S) 247-256 , XP002535515 ISSN: 0022-1554 cité dans la demande le document en entier

## Description

### Domaine technique

La présente invention concerne le domaine des automates de dénombrement et de caractérisation de particules en suspension dans un milieu liquide, et plus particulièrement celui des appareils d'hématologie permettant de dénombrer et de caractériser les différents types de cellules contenues dans un échantillon de sang.

Elle concerne en particulier mais de manière non limitative un procédé de comptage et de classification des globules blancs.

La présente invention concerne également un dispositif mettant en oeuvre le procédé.

### Etat de la technique antérieure

Les globules blancs normaux sont classés en cinq types: Monocytes, Lymphocytes, Neutrophiles, Éosinophiles et Basophiles. Le nombre total de globules blancs et leur répartition relative entre ces cinq sous-populations permet d'établir ou de guider le praticien vers une pathologie.

L'état de l'art et l'invention proposée concernent donc les dispositifs permettant de compter le nombre de globules blancs contenus dans un échantillon de sang et de déterminer leur répartition relative entre les cinq sous-populations.

On connaît le document US 2,656,508 de W.H. Coulter et al., qui décrit un procédé de comptage absolu de particules en suspension par mesure d'impédance. Selon ce procédé, le nombre de particules par unité de volume est déterminé par une technique d'impédancemétrie qui consiste à mesurer la variation d'impédance d'un micro-orifice lorsqu'une cellule sanguine le traverse. Cette technique, simple et robuste, ne requiert pas de dispositifs fluidiques complexes et est librement utilisable puisque que le brevet US 2,656,508 qui la protégeait date de 1953. Elle est donc systématiquement exploitée par la totalité des fabricants d'automates (Beckman Coulter, Abbott, Bayer, C2 Diagnostics, Sysmex, ABX,...) pour le comptage absolu du nombre de globules blancs.

Il est connu que dans cette technique d'impédancemétrie, la forme d'une impulsion générée par le passage d'une cellule dépend de la géométrie du micro-orifice et de la trajectoire de la cellule dans celle-ci. On connaît par exemple l'article de Von Behrens et al. (Von Behrens, J. of Histochemistry and Cytochemistry, 1976, Vol.24, No.1, p.247) qui décrit la géométrie des lignes du champ électrique généré dans un orifice de section rectangulaire ou circulaire, et la forme des impulsions résistives en fonction de la trajectoire de la cellule ou de la particule dans cet orifice. La plupart des dispositifs de l'art antérieur essaient de minimiser cette dépendance de la forme de l'impulsion résistive avec la trajectoire en optimisant soit la géométrie de l'orifice, tel que décrit par exemple par T. Zhao et al. dans le document US 2008/0093216, soit la disposition et la structure des électrodes, tel que décrit par exemple dans les documents et US 4,420,720 de Newton et al. et US 2001/0052763 de North et al., respectivement.

La technique d'impédancemétrie seule ne permet toutefois pas de différentier de façon fiable les cinq sous-populations de globules blancs. Cette différentiation des globules blancs selon leurs cinq sous-populations est avantageusement réalisée en combinant l'impédancemétrie avec des techniques de cytométrie en flux optiques. On connaît le document US 5,812,419 de V.L. Chupps et al. qui décrit un procédé d'analyse automatique des cellules sanguines combinant, outre l'impédancemétrie, des mesures optiques d'absorption, de spectrophotométrie, de diffraction à différents angles et de fluorescence. Selon ce procédé, les mesures par impédancemétrie et cytométrie en flux optique sont réalisées dans des cuves d'analyses différentes, et des algorithmes de fusion de données permettent d'obtenir le comptage et les caractérisations de cellules voulues. Le procédé décrit par Chupps et al. montre bien l'intérêt de l'usage combiné de l'impédancemétrie et de la cytométrie de flux optique, mais l'usage de cuves d'analyses différentes rend le système très complexe. De plus, la possible inhomogénéité des échantillons de sang analysés par les différentes méthodes est une source non négligeable d'incertitude de mesure.

Dans le document US 6,228,652 C.M. Rodriguez et al. décrivent une cuve de cytométrie permettant de faire à la fois des mesures d'impédance et optiques sur les mêmes cellules, aboutissant ainsi à une simplification du dispositif et une réduction des problèmes de corrélation de mesures entre transducteurs multiples. Le dispositif selon US 6,228,652 permet ainsi de mesurer pour chaque cellule son volume, qui est fonction de sa résistivité à tension continue, son opacité radiofréquence qui est fonction de sa conductivité à la fréquence électrique de mesure, et ses caractéristiques de diffraction et de fluorescence optiques.

La simplification des dispositifs est un enjeu industriel de taille dans le développement des automates pour l'hématologie, dans la mesure où elle ouvre des marchés considérables dans les petites structures de soins et les pays émergents. On connaît par exemple le document FR 2 653 885 de D. Lefevre et al. qui décrit un dispositif de cytométrie optique et électrique dans laquelle les électrodes pour la mesure résistive sont des parties constitutives du dispositif de transfert du flux de cellules.

La cytométrie en flux optique impose des contraintes importantes en termes de positionnement relatif du flux contenant les cellules et des moyens d'éclairement. En effet, il est important pour la qualité des mesures optiques que les cellules traversent le faisceau dans une zone, dite zone de mesure optique, où l'éclairement puisse être considéré comme suffisamment uniforme en intensité. Une cellule traversant le faisceau dans une zone où l'éclairement est incorrect peut engendrer une mesure incorrecte et une erreur de détermination du type de cellule. La difficulté consiste donc à contraindre le flux transportant les cellules à circuler de façon à ce qu'il soit entièrement à l'intérieur de la zone de mesure optique d'intensité uniforme.

La solution généralement mise en oeuvre dans les dispositifs de l'art antérieur inclut l'utilisation d'un ou plusieurs flux de gainage, appelés aussi manchons, qui circulent de manière concentrique avec le flux central transportant les cellules. Ces flux de gainage étirent le flux central, et confinent ainsi de manière très précise les particules qu'il contient. Ainsi, FR 2 653 885 par exemple met en oeuvre une technique de double manchonnage selon laquelle le flux central est confiné par deux flux de gainage concentriques. La génération des flux de gainage et du flux central transportant les cellules sanguines requiert de multiples entrées fluidiques ainsi que des micro-buses. Cela conduit à des ensembles de composants fluidiques particulièrement denses autour de la zone de mesure, rendant l'assemblage des capteurs délicat et coûteux. De plus, pour que la localisation des particules par le flux de gainage soit efficace, il faut que le débit du flux de gainage soit nettement plus important que le débit du flux contenant l'échantillon. Cela signifie qu'un volume important du réactif mis en oeuvre, généralement un diluant, n'a pas de d'utilité chimique mais est seulement utilisé pour guider le flux échantillon à mesurer. L'impact en termes de consommation de réactifs et de production de déchets est évident.

De plus le taux de dilution utilisé dans ce type de solutions est incompatible avec le taux de dilution nécessaire à la mesure de l'hémoglobine qui est généralement mesurée avec la dilution des globules blancs et qui nécessite donc d'effectuer une dilution supplémentaire ainsi que des éléments fluidiques supplémentaires couteux.

Les documents US 6614215 B1 et WO 01/69202 A2 décrivent des dispositifs de détection de particules mettant en oeuvre une mesure d'impédance et une mesure de fluorescence.

Le document CH 544305 A montre un procédé permettant de détecter le passage de particules au travers d'une ouverture par mesures d'impédance, et de discriminer les particules qui ne suivent pas un trajet axial dans la cuve.

Le but de la présente invention est de proposer un procédé de cytométrie en flux comprenant des mesures d'impédance et des mesures optiques qui ne nécessite pas de flux de manchonnage, permettant ainsi une simplification considérable de la cuve ou cellule de mesure et des éléments fluidiques associés.

Le but de la présente invention est également de proposer un dispositif mettant en oeuvre le procédé, plus simple et plus économique tant à fabriquer qu'à entretenir que les dispositifs de l'art antérieur, permettant l'utilisation d'automates ainsi équipés par des laboratoires de moindre importance tout en conservant une qualité de mesure équivalente.

### Exposé de l'invention

Cet objectif est atteint avec un procédé de cytométrie en flux pour la caractérisation de particules selon la revendication 1 comprenant des étapes de :
- transfert d'un fluide contenant des particules au travers d'une cuve-orifice, laquelle cuve-orifice étant au moins en partie sensiblement transparente à au moins une longueur d'onde d'intérêt, et au moins en partie sensiblement isolante à l'électricité,
- mesure de variation d'impédance électrique générée par le passage desdites particules contenues dans le fluide au travers de la cuve-orifice,
- mesure d'au moins une propriété optique desdites particules, lesquelles interagissant avec au moins un faisceau de lumière traversant l'orifice de la cuve-orifice dans une zone de mesure optique,
- caractérisation desdites particules en exploitant lesdites mesures de variation d'impédance électrique et de propriétés optiques,
caractérisé en ce qu'il comprend en outre des étapes de :
- génération, au moyen d'une diode électroluminescente, d'un faisceau de taille supérieure au diamètre de l'orifice,
- déduction d'information sur la trajectoire des particules dans la cuve-orifice, au moins à partir de l'analyse de ladite mesure de variation d'impédance électrique,
- utilisation de ladite information sur la trajectoire au moins pour l'exploitation desdites mesures de propriétés optiques lors de la caractérisation des particules en classant lesdites mesures en au moins deux catégories dites valides et non valides.

Suivant des caractéristiques particulièrement avantageuses,
- le procédé selon l'invention est un procédé de cytométrie en flux qui ne nécessite pas l'utilisation de flux de gainage ou de manchonnage comme dans les procédés de l'art antérieur. Le principe du confinement physique des particules par les fluides de manchonnage est remplacé par la possibilité d'effectuer un traitement sélectif des données issues des mesures sur ces particules, lequel traitement étant conditionné ou déterminé par de l'information sur leur trajectoire ;
- seul le fluide contenant les particules est transféré au travers de la cuve-orifice ;
- la cuve-orifice peut être considérablement simplifiée en comparaison des dispositifs de l'art antérieur. Elle peut se réduire à une simple ouverture ou orifice dans la paroi d'une cuve, au travers duquel le fluide contenant les particules est transféré et dans lequel les mesures sont effectuées.

Avantageusement,
- la mesure de variation d'impédance électrique peut comprendre une mesure de résistivité effectuée en courant continu ;
- la mesure de variation d'impédance électrique peut également comprendre au moins une mesure d'impédance complexe effectuée à au moins une fréquence non nulle ;
- l'au moins une propriété optique mesurée peut être une des propriétés optiques suivantes : absorbance, diffusion élastique selon au moins un angle, diffusion inélastique selon au moins un angle, rétrodiffusion élastique, rétrodiffusion inélastique, autofluorescence, fluorescence à au moins une longueur d'onde provoquée par au moins un marqueur greffé sur les particules, fluorescence à au moins une longueur d'onde provoquée par au moins un marqueur greffé à au moins un élément contenu dans les particules, polarisation de la lumière ;
- la caractérisation de particules peut comprendre une classification des particules ;
- la caractérisation de particules peut également comprendre un dénombrement des particules ;
- l'analyse de la variation d'impédance électrique peut comprendre une comparaison entre la variation d'impédance électrique mesurée et au moins un élément de comparaison parmi au moins un modèle de variation d'impédance électrique et au moins une valeur numérique préalablement définie, le résultat de ladite comparaison déterminant si la trajectoire des particules a traversé une zone de la cuve-orifice préalablement définie ;
- les informations relatives aux particules dont la trajectoire n'a pas traversé une zone de la cuve-orifice préalablement définie peuvent être retirées des données utilisées pour la classification desdites particules. Ainsi, il est possible dans le procédé selon l'invention de trier les particules en fonction de leur trajectoire, par exemple pour ne conserver les informations que de celles qui satisfont certaines conditions ;
- la propriété optique mesurée des particules peut également être comparée avec au moins un élément de comparaison parmi au moins un modèle de propriété optique et au moins une valeur numérique préalablement définie. Dans ce cas, si les résultats des comparaisons de la variation d'impédance électrique et de la propriété optique avec leurs modèles ou valeurs numériques préalablement définies respectifs ne satisfont pas au moins un critère préalablement défini, les informations relatives aux particules peuvent être retirées des données utilisées pour la classification desdites particules ;
- l'opération de classification des particules peut comprendre des étapes d'attribution aux particules d'un ensemble de valeurs ou coordonnées issues de l'analyse desdites mesures effectuées lors de leur passage dans la cuve-orifice, lesquelles coordonnées définissant leur position dans un espace de représentation préalablement défini, et des étapes de segmentation dudit espace de représentation en régions distinctes, lesquelles régions regroupant des particules de caractéristiques sensiblement similaires ;
- les particules peuvent comprendre des cellules sanguines. Ces cellules sanguines peuvent être des leucocytes.

Suivant un autre aspect de l'invention, il est proposé un dispositif de cytométrie en flux pour la caractérisation de particules selon la revendication 12 comprenant :
- une cuve-orifice au moins en partie sensiblement transparente à au moins une longueur d'onde d'intérêt, et au moins en partie sensiblement isolante à l'électricité,
- des moyens de transfert d'un fluide contenant des particules au travers de ladite cuve-orifice,
- des moyens de mesure de variation d'impédance électrique générée par le passage desdites particules contenues dans le fluide au travers de la cuve-orifice,
- des moyens de mesure d'au moins une propriété optique desdites particules, lesquelles particules interagissant avec au moins un faisceau de lumière traversant l'orifice de la cuve-orifice dans une zone de mesure optique,
- des moyens d'analyse desdites mesures de variation d'impédance électrique et de propriétés optiques,
caractérisé en ce qu'il comprend :
- une diode électroluminescente pour générer un faisceau de taille supérieure au diamètre de l'orifice,
- des moyens de déduction d'information sur la trajectoire des particules dans la cuve-orifice, au moins à partir de l'analyse de ladite mesure de variation d'impédance électrique, et
- des moyens d'utilisation de ladite information sur la trajectoire au moins pour l'exploitation desdites mesures de propriétés optiques lors de la caractérisation des particules en classant lesdites mesures en au moins deux catégories dites valides et non valides.

Le dispositif selon l'invention peut comprendre en outre des moyens de préparation des particules d'intérêt avant leur transfert au travers de la cuve-orifice. Les particules d'intérêt sont les particules que l'on souhaite analyser et classifier, et qu'il peut être nécessaire de séparer d'autres particules présentes dans l'échantillon, notamment par des procédés de lyse ou de dilution dans le cas de cellules sanguines.

Avantageusement,
- la géométrie de la cuve-orifice peut être adaptée de telle sorte que la variation d'impédance électrique mesurée lors du passage des particules dépende de manière sensible de leur trajectoire dans ladite cuve-orifice. Ce résultat peut être obtenu par exemple avec une cuve-orifice de section sensiblement rectangulaire dont les côtés longitudinaux se terminent sur des angles à faible rayon de courbure. Il est à noter qu'on recherche ici un résultat à l'opposé de la pratique dans les dispositifs de l'art antérieur dans lesquels on cherche plutôt à minimiser cette dépendance de la trajectoire des particules ;
- la cuve-orifice peut comprendre une section transversale interne sensiblement de l'une des formes suivantes : circulaire, rectangulaire ;
- la cuve-orifice peut être constituée de l'un au moins des matériaux suivants : saphir, rubis, verre, plastique ;
- les moyens de mesure de variation d'impédance électrique générée par le passage des particules peuvent comprendre au moins deux électrodes disposées respectivement de part et d'autre de l'orifice de la cuve-orifice, lesquelles électrodes étant en contact électrique avec le fluide contenant les particules ;
- les moyens de mesure de variation d'impédance électrique générée par le passage des particules peuvent également comprendre au moins une électrode disposée dans l'orifice de la cuve-orifice, laquelle électrode étant en contact électrique avec le fluide contenant les particules. Un dispositif suivant l'invention peut ainsi comprendre par exemple des électrodes distribuées le long de la paroi de la cuve-orifice. L'une au moins des électrodes peut également être un élément constitutif des moyens de transfert du fluide contenant les particules ;
- les moyens de mesure de la propriété optique des particules peuvent comprendre au moins une source de lumière, et au moins un moyen de détection optoélectronique ;
- les moyens de mesure de la propriété optique des particules peuvent également comprendre au moins un moyen optique de focalisation de la lumière issue de la source de lumière dans la cuve-orifice ;
- les moyens de mesure de la propriété optique des particules peuvent également comprendre au moins un moyen optique de collecte de la lumière issue de la cuve-orifice ;
- les moyens de mesure de la propriété optique des particules peuvent en outre comprendre au moins un moyen de filtrage spectral de la lumière provenant des particules à caractériser ;
- au moins un desdits moyen optique de mesure de la propriété optique des particules peut être intégré à la cuve-orifice. Une telle intégration est par exemple possible en mettant en oeuvre une cuve-orifice moulée en matière plastique, dont les parois externes constitueraient des lentilles de focalisation et éventuellement de collecte de la lumière. Des éléments optiques tels qu'un détecteur optoélectronique par exemple peuvent être également directement collés sur la cuve-orifice.

Suivant un mode de réalisation particulier, le moyen de détection optoélectronique peut être disposé de telle sorte à mesurer un faisceau lumineux provenant des particules à caractériser qui présente une orientation angulaire différente de celle de l'axe du faisceau issu de la source de lumière. Une telle configuration permet par exemple de faire des mesures de diffusion à des angles non nuls.

Suivant un autre mode de réalisation particulier, les moyens de mesure de la propriété optique des particules peuvent comprendre une source de lumière présentant un état de polarisation connu, et des moyens de détection optoélectronique comprenant des moyens d'analyse de la polarisation de la lumière.

Suivant encore un autre aspect de l'invention, il est proposé un appareil d'analyse sanguine comprenant au moins un dispositif selon l'invention, les particules à analyser étant des cellules sanguines, et notamment des leucocytes.

### Description des figures et modes de réalisation

D'autres avantages et particularités de l'invention apparaîtront à la lecture de la description détaillée de mises en oeuvre et de modes de réalisation nullement limitatifs, et des dessins annexés suivants :
- La figure 1 présente un schéma du dispositif selon l'invention ;
- La figure 2 illustre l'influence du positionnement des particules dans le faisceau de lumière en cytométrie de flux. La figure 2a représente un cas où le flux des particules n'est pas centré dans le faisceau, tandis que dans la figure 2b le flux de particules est centré dans le faisceau. L'effet est illustré en prenant le cas, nullement limitatif, de mesures d'absorbances ;
- Les figures 3a et 3b illustrent la déviation d'un faisceau de lumière par la surface d'un orifice cylindrique, et la délimitation des zones de mesure optiques fiables qui en découlent ;
- La figure 4a présente un exemple de forme de lignes équipotentielles apparaissant dans une ouverture microscopique faite dans un matériau isolant, lorsqu'on applique une tension au travers de cette ouverture. La figure 4b illustre la forme des impulsions résistives obtenues lorsque des particules traversent cette ouverture, selon diverses trajectoires. Ces illustrations sont reprises de V. Kachel, Flow cytometry and Sorting, Wiley-Liss Inc., 1990, également cité dans US 2008/0093216 ;
- La figure 5a présente des exemples de trajectoires de particules à l'intérieur d'une cuve-orifice de forme cylindrique, ainsi que la délimitation de la zone de mesure optiques fiable telle que définie à la figure 3. La figure 5b présente la forme des impulsions résistives qui seraient obtenues pour des particules parcourant les trajectoires illustrées à la figure 5a ;
- La figure 6 présente un organigramme de l'acquisition et du traitement des données selon un mode de mise en oeuvre préférentiel du procédé selon l'invention ;
- La figure 7 présente un chronogramme de l'acquisition et du traitement des données conformément à l'organigramme de la figure 6 ;
- La figure 8 présente des exemples de signaux mesurés correspondant respectivement à des impulsions résistives et à des impulsions optiques issues de mesures d'absorbance. Ces signaux sont représentatifs, respectivement, des trajectoires T1 et T4 telles que présentées à la figure 5 ;
- La figure 9 présente des exemples de diagramme de classification de particules, ici des leucocytes, obtenus avec un dispositif selon l'invention. La figure 9a illustre un cas où toutes les particules détectées sont représentées. A la figure 9b ne sont représentées que les particules triées conformément au procédé selon l'invention ;
- La figure 10 illustre un mode de réalisation, préférentiel mais nullement limitatif, de dispositif selon l'invention.
- La figure 11 présente un schéma d'un dispositif de l'art antérieur mettant en oeuvre un confinement des particules dans la zone de mesure optique au moyen de fluides de gainage ;

On va décrire en référence aux figures un mode de réalisation préférentiel mais nullement limitatif du dispositif mettant en oeuvre le procédé selon l'invention dans un appareil permettant de dénombrer et de caractériser les différents types de cellules contenus dans un échantillon de sang, et en particulier les globules blancs. Cet appareil peut permettre par exemple de déterminer le nombre total de globules blancs et leur répartition relative entre leurs cinq sous-populations : Monocytes, Lymphocytes, Neutrophiles, Éosinophiles et Basophiles.

Dans la suite de la description, les particules sont assimilées à des globules blancs ou leucocytes sans que cela ne présente un quelconque caractère limitatif.

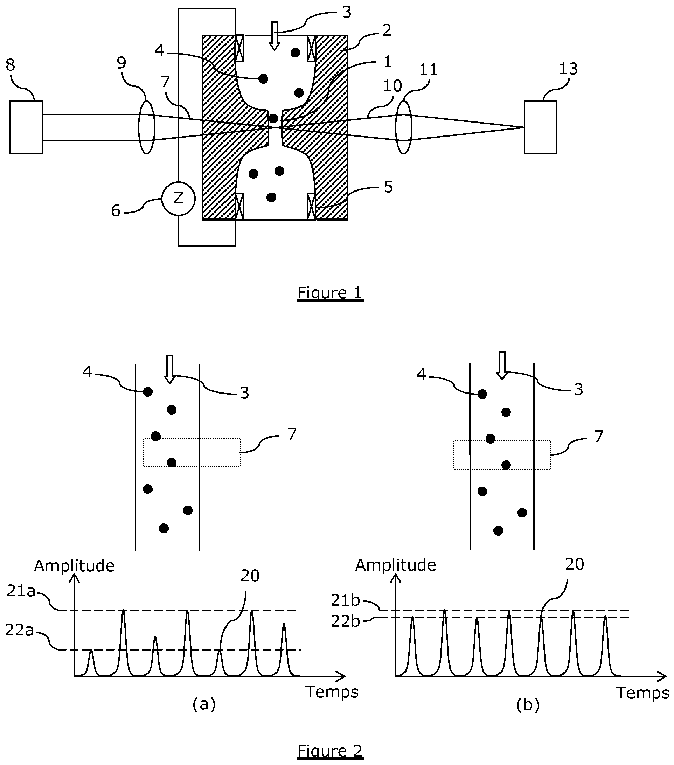

En référence à la figure 1, le dispositif mettant en oeuvre le procédé selon l'invention comprend une cuve-orifice 2 au travers de laquelle un fluide 3 contenant des particules 4 est transféré. Cette cuve-orifice comprend un orifice 1 où ont lieu principalement les interactions entre les particules et les moyens de mesure. Le transfert du fluide 3 au travers de la cuve-orifice 2 peut être effectué par différentes techniques sans sortir du champ de l'invention, parmi lesquelles on peut citer notamment l'écoulement par gravité, l'aspiration, et l'injection en pression.

De préférence, la cuve-orifice 2 et l'orifice 1 ont une section transverse sensiblement circulaire, et l'orifice 1 a un diamètre de l'ordre de 80 µm. Ces spécifications sont bien entendu nullement limitatives, et un dispositif selon l'invention peut tout aussi bien comprendre une cuve-orifice 2 de section sensiblement rectangulaire, ou polygonale, d'une autre dimension.

Afin de permettre la mise en oeuvre des mesures d'impédance et des mesures optiques, la cuve-orifice 2 doit être constituée au moins en partie d'au moins un matériau sensiblement isolant à l'électricité et au moins en partie d'au moins un matériau sensiblement transparent aux longueurs d'ondes optiques d'intérêt. Suivant un mode de réalisation préférentiel, elle est réalisée dans du saphir. Il est bien entendu possible sans sortir du champ de l'invention de mettre en oeuvre une cuve-orifice 2 réalisée dans un autre matériau tel que du rubis, un verre, un polymère ou un plastique, ou même réalisée avec une combinaison de plusieurs matériaux.

Suivant un mode de réalisation préférentiel, des électrodes 5 sont disposées de part et d'autre de la cuve-orifice 2. Elles sont en contact électrique avec le fluide, de telle sorte à permettre l'établissement d'un champ électrique longitudinal dans la cuve-orifice et en particulier au travers de l'orifice 1. Ces électrodes 5 sont reliées à un dispositif 6 qui mesure les variations d'impédance lors du passage des particules 4 dans l'orifice 1 de la cuve-orifice 2, c'est-à-dire la forme temporelle et l'amplitude des impulsions dues au passage de ces particules. La mesure d'impédance mise en oeuvre dans le dispositif selon l'invention est de préférence mais de manière non limitative une mesure de résistivité effectuée à courant continu.

Suivant un mode de réalisation préférentiel, le dispositif selon l'invention comprend au moins un dispositif de mesures optiques, dont le principe général est de mesurer le résultat d'une interaction entre une particule telle qu'un leucocyte et un faisceau de lumière. Cette mesure est également appelée mesure d'une propriété optique de la particule.

En référence à la figure 1, un tel dispositif de mesure optique peut comprendre notamment une source de lumière 8, et au moins un moyen de détection 13. Il peut également comprendre au moins un moyen optique de focalisation 9 du faisceau 7 dans la cuve-orifice 2, et au moins un moyen optique de collecte 11 de la lumière issue de la cuve-orifice 2.

La source de lumière 8 peut être par exemple un laser, une diode laser, ou une diode électroluminescente.

Les moyens optiques de focalisation 9 et de collecte 11 peuvent comprendre notamment des lentilles sphériques, asphériques, cylindriques, de forme libre (« freeform optics » en anglais), à gradient d'indices et/ou des surfaces réfléchissantes (miroirs). Ils peuvent également comprendre au moins un guide de lumière, lequel orientant par exemple les faisceaux issus de la zone de mesure vers la surface sensible du moyen de détection 13.

Le moyen de détection 13 peut comprendre au moins un photodétecteur tel qu'une photodiode, une photodiode à avalanche ou un tube photomultiplicateur.

Avantageusement, au moins un parmi les moyens optiques de focalisation 9 et de collecte 11 peut être au moins en partie intégré à la cuve-orifice 2, par exemple par collage ou en adaptant la forme extérieure de cette dernière de telle sorte à constituer la surface d'une lentille. Il est également possible de fixer le moyen de détection 13 directement sur la cuve-orifice 2, éventuellement sans mettre en oeuvre de moyens de collecte 11.

Selon un mode de réalisation préférentiel mais nullement limitatif du dispositif selon l'invention, il est mis en oeuvre une mesure d'absorbance suivant laquelle on mesure une variation d'intensité lumineuse sur le détecteur 13 lorsqu'un leucocyte traverse le faisceau de lumière 7. C'est donc la mesure de cette propriété optique qui sera développée dans la suite de la description. L'homme du métier pourra toutefois aisément mettre en oeuvre le procédé selon l'invention dans des dispositifs mettant en oeuvre la mesure d'autres propriétés optiques de la particule, et ceci sans sortir du cadre de l'invention.

Avantageusement, suivant ce mode de réalisation,
- la source de lumière 8 peut être une diode électroluminescente émettant dans le bleu ;
- le dispositif optique de focalisation 9 peut être constitué d'une lame à face parallèle orientable permettant de centrer le faisceau 7 sur la cuve-orifice 2, et d'un doublet achromatique et d'une lentille cylindrique pour focaliser le faisceau 7 dans la cuve-orifice 2, laquelle étant dans le mode de réalisation préférentiel sensiblement cylindrique ;
- le dispositif optique de collecte 11 peut être constitué de deux lentilles plan convexe imageant la zone d'interaction de la lumière avec les particules 4 sur les moyens de détection 13, lesquels comprenant par exemple une photodiode.

Pour obtenir des résultats fiables, il faut que la zone de mesure optique où ont lieu les interactions entre les particules et la lumière ait des dimensions limitées, par exemple de l'ordre de 100µm x 30µm, et que la distribution en intensité de la lumière incidente y soit sensiblement homogène. La difficulté consiste à contraindre le flux transportant les particules à circuler de façon à ce qu'il passe entièrement à l'intérieur de cette zone de mesure optique. Cette problématique est illustrée par la figure 2 dans le cas, non limitatif, de mesures d'absorbance sur des particules présentant une absorbance sensiblement identique. Lorsque la totalité du flux 3 de particules 4 traverse la zone de mesure optique 7, les pics d'absorbance 20 ont tous sensiblement la même amplitude, entre 21b et 22b. Si le flux 3 transportant les particules vient en limite de la zone de mesures optiques 7 ou même dépasse de celle-ci comme illustré à la figure 2a, les particules 4 ne reçoivent plus une quantité égale de lumière et la mesure optique se trouve entachée d'erreur. Cette erreur se traduit par une forte variabilité, entre 21a et 22a, de l'amplitude des pics 20 qui se traduit à son tour par une incertitude accrue dans la classification des particules.

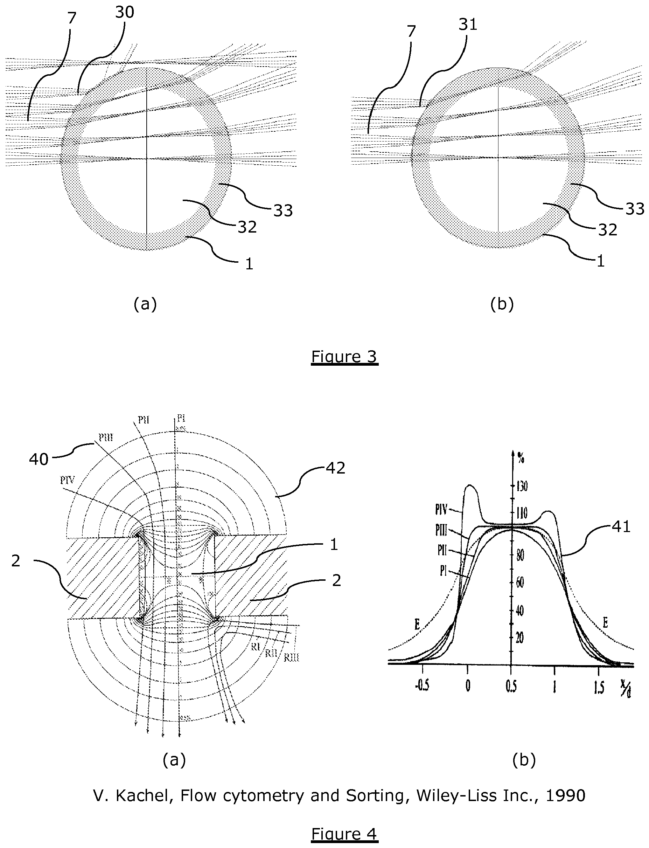

La figure 3 illustre la déviation d'un faisceau de lumière 7 par la surface d'un orifice cylindrique tel que l'orifice 1 de la cuve-orifice 2. Dans la figure 3a, le faisceau est de taille supérieure au diamètre de l'orifice 1. Du fait de l'angle d'incidence sur la surface interne de l'orifice 1, certaines parties 30 de ce faisceau 7 sont réfléchies ou au moins fortement réfracté avec pour conséquence qu'une zone périphérique 33 de l'orifice 1 n'est pas soumise à un éclairage suffisamment homogène. Dans la figure 3b, le faisceau est de taille inférieure au diamètre de l'orifice 1. De même, une zone périphérique 33 de l'orifice 1 située au-delà du rayon limite 31 ne reçoit pas un éclairage correct. Dans les deux cas, seules les particules étant passées par la zone de mesure optique dite fiable 32 seront mesurées dans de bonnes conditions. Celles qui passent dans la zone 33 peuvent donner des résultats entachés d'erreurs. Ce raisonnement peut bien entendu aisément être étendu par l'homme du métier à d'autres géométries d'orifices 1 et de cuves-orifice 2 sans sortir du cadre de l'invention.

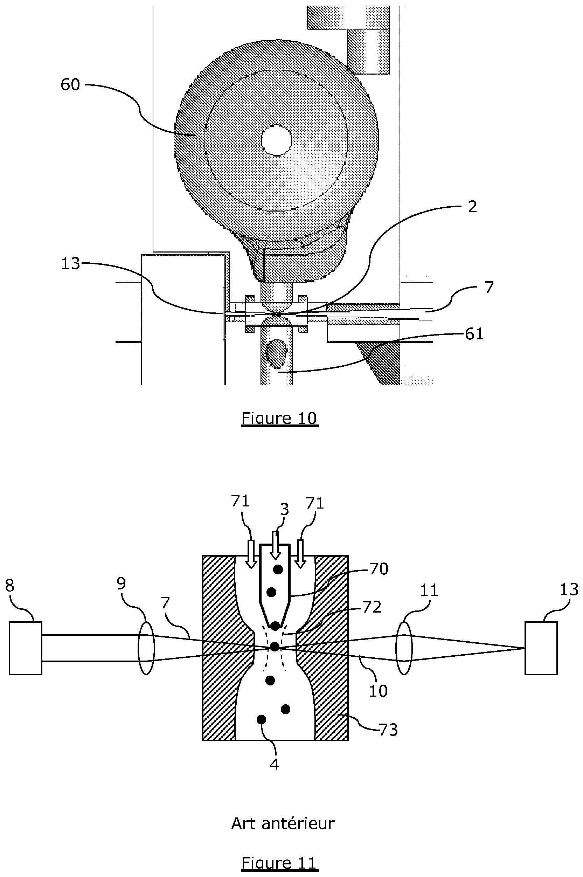

La solution généralement retenue dans les dispositifs de l'art antérieur, telle que présentée à la figure 11, comprend un confinement 72 du flux 3 transportant les particules 4 au centre de la cuve de mesure 73, au moyen d'un ou plusieurs flux de gainage concentriques 71, avec tous les inconvénients déjà évoqués en termes de complexité, de coût de fabrication et de coût d'exploitation, notamment du fait de l'utilisation d'injecteurs 70.

La solution retenue dans le procédé selon l'invention comprend au contraire une identification des particules dont la trajectoire dans la cuve-orifice ne passe pas par une zone prédéterminée, par exemple la zone de mesures optique fiable 32, de telle sorte à pouvoir les traiter différemment dans l'exploitation des données. Cette identification peut être effectuée notamment en analysant la forme des impulsions issues de la mesure d'impédance. En d'autres termes, l'information sur la trajectoire des particules 4 dans l'orifice 1 de la cuve-orifice 2 déduite notamment de l'analyse des variations d'impédance électrique est exploitée pour conditionner l'opération de caractérisation des particules, c'est-à-dire modifier la façon dont les mesures sont exploitées.

En référence à la figure 4, lorsqu'une particule telle qu'un leucocyte traverse une ouverture dans laquelle est appliqué un champ électrique, la variation temporelle de la résistivité au travers de cette ouverture, qui peut être l'orifice 1 de la cuve-orifice 2 du dispositif selon l'invention, prend la l'allure d'une impulsion 41 dont la forme dépend sensiblement de la trajectoire 40 de la particule. En particulier on voit sur l'exemple de la figure 4 que cette impulsion 41 s'élargit et finit par présenter un plateau puis des rebonds à mesure que la trajectoire de la particule s'éloigne du centre de la cuve-orifice 2. Cet effet est dû à la forme du champ électrique dont les lignes équipotentielles 42 autour et dans l'ouverture 1 sont illustrées à la figure 4a.

Cet effet, qui est considéré dans les dispositifs de l'art antérieur comme une source d'erreur à minimiser, est avantageusement exploité dans la présente invention. Il permet de caractériser les trajectoires de particules dans la cuve-orifice à partir de l'analyse de la forme des impulsions résistives, laquelle forme pouvant être considérés comme une signature de ces trajectoires.

La disposition et la surface des électrodes 5 de part et d'autre de la cuve 2 ainsi que la géométrie de cette cuve 2 peuvent même être avantageusement étudiées afin que la forme de l'impulsion 41 ait une dépendance forte de la trajectoire de la particule, ce qui va à l'encontre des conceptions mises en oeuvre dans les dispositifs de l'art antérieur. Toutefois, un orifice 1 de forme cylindrique se terminant sur des angles à faible rayon de courbure, tel que mis en oeuvre dans un mode de réalisation préférentiel, présente déjà les caractéristiques nécessaires.

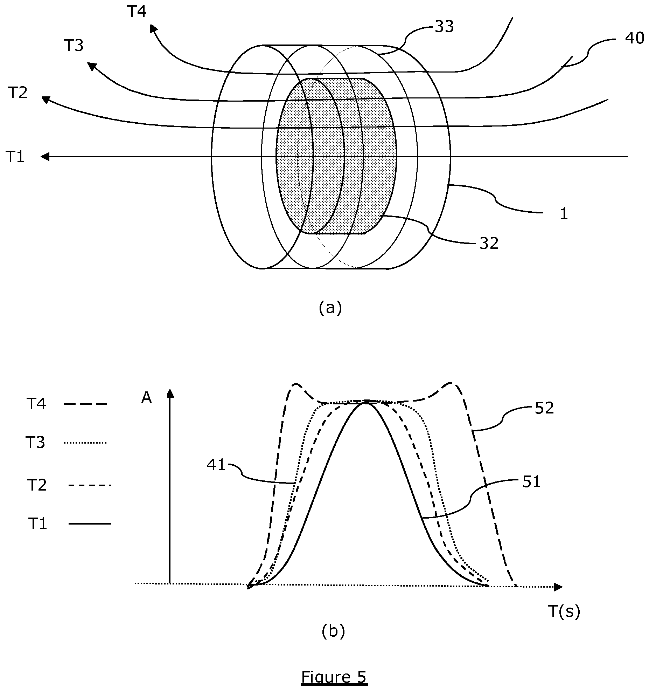

Un exemple de signatures représentatives de trajectoires de particules dans une cuve-orifice est présenté à la figure 5. Dans cet exemple la zone de mesure optique se présente sous la forme d'un cylindre qui correspond à l'intérieur de l'orifice 1 de la cuve-orifice 2, laquelle pouvant être également de forme sensiblement cylindrique. La zone de mesure optique fiable 32 est représentée par un cylindre circonscrit dans la zone de mesure optique totale, et la signature des trajectoires optimales T1 de particules traversant cette zone de mesure optique fiable 32 est de forme sensiblement gaussienne 51. Cette signature présente un plateau de plus en plus large au fur et mesure que la trajectoire s'éloigne du centre de l'orifice (trajectoires T2, T3, T4). Il est dont possible d'associer à la limite de la zone de mesure optique fiable 32 une forme d'impulsion ou une signature limite et de déterminer par rapport à un critère mathématique si une trajectoire est valide (signature 51) ou non valide (signature 52). L'évolution de la forme des impulsions résistives tel qu'illustrée à la figure 5 est bien entendu un exemple non-limitatif, leur comportement pouvant différer selon la géométrie de la cuve-orifice et aussi selon le type de particules.

Selon le mode de réalisation préférentiel, le critère mathématique appliqué pour déterminer la validité d'une trajectoire est issu de la comparaison des impulsions mesurées avec au moins une valeur limite de largeur d'impulsion pour au moins une hauteur de ladite impulsion.

Suivant un mode de réalisation préférentiel mais non limitatif dans lequel est mis en oeuvre une mesure de résistivité et une mesure d'absorbance optique, les particules traversant la cuve-orifice génèrent donc deux impulsions, ou signaux électriques : une impulsion résistive issue du capteur d'impédancemétrie et une impulsion issue du capteur d'absorbance optique.

Les impulsions optiques sont conditionnées analogiquement, amplifiées et filtrées selon les techniques usuelles pour être transmises au convertisseur analogique/numérique.

Les impulsions résistives sont obtenues par des techniques classiques mais un soin particulier est apporté à la mise en oeuvre du capteur afin de maximiser le rapport signal sur bruit et de disposer des informations suffisantes à la caractérisation et au tri, tel que le choix de la bande passante.

Ces signaux issus des capteurs d'impédancemétrie et d'absorbance optique sont conditionnés et transmis à un convertisseur analogique/numérique qui est associé à un système de traitement numérique pouvant inclure notamment un microprocesseur et/ou un FPGA et/ou un DSP. Le taux d'échantillonnage des impulsions optiques et résistives est adapté à chaque type de signal de façon à obtenir le niveau d'information nécessaire au traitement numérique.

Le traitement des impulsions optiques peut être limité à l'extraction des informations d'amplitude et de largeur.

Le signal échantillonné issu du capteur résistif est mis en forme au moyen des techniques de traitement du signal classiques basées sur les caractéristiques temporelles et fréquentielles du signal à analyser telles que les filtrages numériques à réponse impulsionnelle finie ou infinie, le filtrage optimal, la transformée de Fourier rapide etc.

On peut ainsi créer un couple formé de l'impulsion résistive et de l'impulsion optique associée, et mémoriser leurs caractéristiques telles que par exemple les amplitudes, les largeurs d'impulsion, etc.

Les impulsions résistives et optique, nettoyées du bruit de fond et normalisées peuvent alors être traité par l'algorithme de tri, lequel classe les mesures en au moins deux catégories, dites valides et non valides.

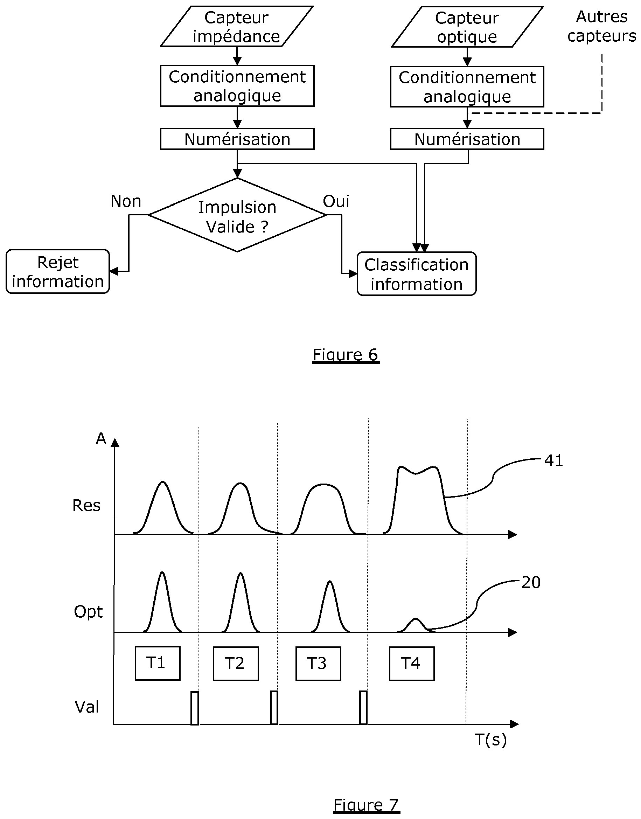

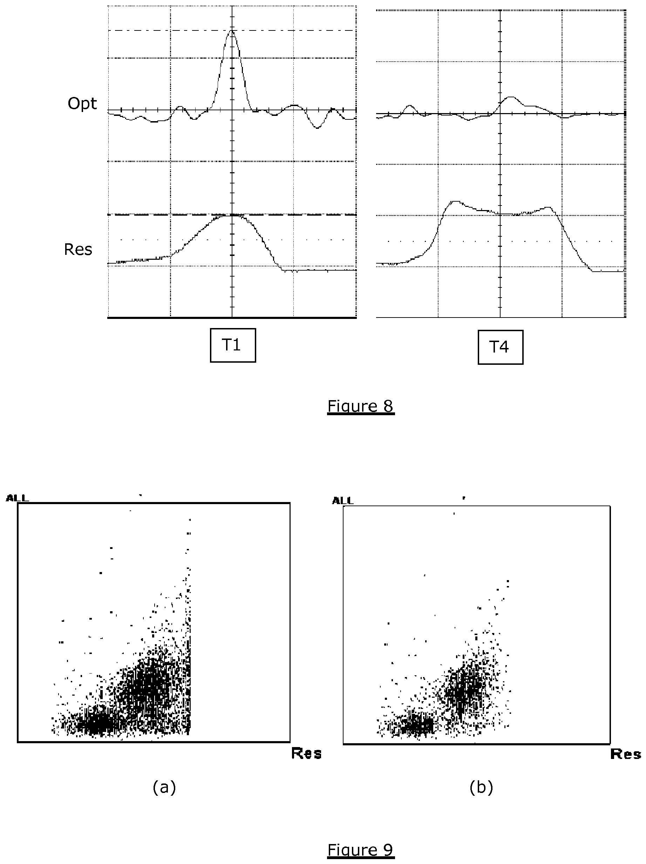

Avantageusement, l'algorithme de tri comprend une mesure de l'écart entre une signature associée à une trajectoire optimale, représentée par une forme d'impulsion résistive optimale, et la signature des trajectoires effectives des particules, c'est-à-dire des l'impulsions résistives mesurées. Si cet écart est trop important les données relatives à ces particules sont considérées comme non valides. La figure 8 présente des exemples de résultat de mesure de couples d'impulsion résistives (Res) et optiques (Opt) valides et non valides. Ils correspondent respectivement à des mesures concernant des particules ayant suivi des trajectoires telles que T1 et T4 de la figure 5. Le chronogramme de la figure 7 illustre la séquence temporelle de l'acquisition et de la classification (Val) des mesures.

L'algorithme de caractérisation, lequel effectue l'analyse des résultats proprement dite, comprend avantageusement au moins une opération de classification des particules en catégories présentant des caractéristiques sensiblement homogènes, et éventuellement au moins une opération de comptage de ces particules. Selon un mode de mise en oeuvre avantageux mais nullement limitatif du procédé, ne sont utilisées pour la classification que les informations relatives aux particules dont les impulsions ou signatures sont classées comme valides, comme illustré sur l'organigramme de la figure 6. Le comptage peut par contre avantageusement être effectué sur la totalité des mesures ayant donné lieu à une impulsion résistive, valide ou non valide. Tout autre mode d'exploitation des données à l'issu du tri des particules peut bien entendu être envisagé sans sortir du cadre de l'invention.

La classification de particules valides, ou selon un mode de réalisation préférentiel, de leucocytes en sous-catégories, peut être avantageusement effectuée en attribuant à ces particules un ensemble de valeurs ou coordonnées issues de l'analyse des mesures résistives et optiques effectuées lors de leur passage dans la cuve-orifice 2, lesquelles coordonnées définissant leur position dans un espace de représentation préalablement défini. Une segmentation de cet espace de représentation en régions distinctes regroupant des particules de caractéristiques sensiblement similaires permet alors d'identifier la population des chacune des sous catégories, pour en mesurer par exemple leur importance relative. La figure 9 présente un exemple de représentation des particules, ici des leucocytes, en fonction des informations issues respectivement de l'analyse des mesures résistives (Res) et d'absorbance optiques (ALL). Chaque point représente un couple de mesures. A la figure 9a sont représentées toutes les mesures, valides et non valides. A la figure 9b ne sont présentées que les mesures valides, ce qui permet de voir apparaître des groupes de points distincts correspondant à des catégories de particules différentes.

Selon une caractéristique avantageuse de l'invention, le procédé et le dispositif peuvent être aisément mis en oeuvre pour réaliser un appareil d'hématologie particulièrement simple. Le dispositif selon l'invention peut être aisément placé au niveau d'un simple bac 60 contenant la solution à analyser, et son orifice associé 61. La cuve-orifice 2 peut être placée à toute position relativement au bac. Cette cuve-orifice peut aussi être placée dans une ligne continue de tubulure et réalisé dans tout type de matériau sensiblement transparent et isolant à l'électricité. Le transfert de la solution à analyser peut par exemple être effectué par aspiration au travers de la cuve-orifice 2. La figure 10 présente un exemple de réalisation, non limitatif, comprenant une seule mesure optique dans l'axe de l'orifice. Plusieurs photodiodes peuvent également être placées autour de l'axe de l'orifice pour permettre des mesures à différents angles et différentes longueurs d'ondes.

Avantageusement cette réalisation permet aussi de combiner aisément une mesure de l'hémoglobine par spectrophotométrie classique au travers du bac translucide contenant la solution à analyser.

Suivant des modes de réalisation particuliers, le dispositif selon l'invention peut comprendre une pluralité d'électrodes réparties en une ou plusieurs couches le long de la cuve-orifice 2, tel que décrit dans le document US 4,420,720 par exemple. Le dispositif selon l'invention peut également comprendre des électrodes de résistivité variable ou non, couvrant tout ou partie de la face interne de la cuve-orifice 2, tel que décrit dans US 2001/0052763 par exemple. Dans le cas d'un dispositif selon l'invention comprenant plus que deux électrodes, il est possible de mettre en oeuvre plusieurs mesures d'impédances entre différents couples d'électrodes.

Suivant des modes de réalisation particuliers, il est possible sans sortir du champ de l'invention de mettre en oeuvre des mesures d'impédances complexes à une ou plusieurs fréquences discrètes, ainsi que dans une ou plusieurs bandes de fréquences électriques continues.

Suivant des modes de réalisation particulier, il est possible de mettre en oeuvre sans sortir du champ de l'invention au moins une mesure d'au moins une propriété optique telle que par exemple :
- une mesure d'autofluorescence, une mesure de fluorescence provoquée par un marqueur greffé sur la membrane des leucocytes, ou une mesure de fluorescence provoquée par un marqueur greffé sur un ou plusieurs éléments du contenu intracellulaire des leucocytes, dans lesquelles on mesure une intensité lumineuse à une longueur d'onde de fluorescence sur au moins un détecteur 13 disposé autour de la cuve-orifice, lequel détecteur étant par exemple isolé de la lumière à la longueur d'onde d'excitation 7 par un filtre spectral ;
- une mesure de diffusion élastique selon au moins un angle, de diffusion inélastique selon au moins un angle, de rétrodiffusion, pour lesquelles notamment un ou plusieurs détecteurs 13 peuvent être disposés autour de la cuve-orifice, selon des orientations différentes de celle de l'axe du faisceau 7, de telle sorte à mesurer la lumière diffusée à différents angles, laquelle pouvant présenter des décalages de fréquence optique ;
- une mesure de polarisation, dans laquelle les moyens de détection 13 sont conçus de telle sorte à analyser la polarisation de la lumière ayant interagi avec la particule, la source 8 ayant une polarisation connue, par exemple linéaire.

Suivant des modes de réalisation particuliers, il est possible dans un dispositif selon l'invention de mettre en oeuvre les mesures de plusieurs propriétés optiques simultanément, par exemple en disposant plusieurs dispositifs de mesure optiques tels que montrés à la figure 1 à différentes orientations ou selon différents axes autour de la cuve-orifice 2, ou en introduisant des moyens de division du faisceau 10 issu de la cuve-orifice.

Suivant des modes de réalisation particuliers, le critère mathématique appliqué pour déterminer la validité d'une trajectoire peut être issu :
- d'au moins une comparaison avec un seuil en amplitude, ou une hauteur minimale d'impulsion,
- d'au moins un calcul de distance au sens mathématique entre une impulsion mesurée et au moins un modèle d'impulsion, laquelle distance étant calculée par exemple au moyen d'une fonction de corrélation normalisée,
- d'au moins une analyse effectuée sur le résultat de l'application d'au moins une transformation telle qu'une transformée de Fourier aux impulsions mesurées,
- ou de toute combinaison de ces méthodes ou toute autre méthode mathématique pertinente, sans sortir du champ de l'invention.

Suivant des modes de réalisation particuliers, il est possible sans sortir du champ de l'invention de définir également un ou plusieurs modèles d'impulsions optiques, de comparer les impulsions optiques mesurées à ce ou ces modèles et d'inclure cette comparaison dans l'algorithme de tri. De même, il est possible de définir un ou plusieurs modèles couplés d'impulsions optiques et résistives et d'effectuer des opérations de comparaison et de tri sur la base de ces modèles. Cela peut permettre de traiter d'autres cas d'erreurs de mesure, dues par exemple à des particules n'appartenant pas aux catégories attendues, ou au fait que plusieurs particules sont mesurées en même temps.

Suivant des modes de réalisation particuliers, le procédé et le dispositif associés peuvent être mis en oeuvre dans un dispositif réalisé sur la base de technologies relevant du domaine des microsystèmes (MEMs), ou des microsystèmes optiques (MOEMs), de telle sorte par exemple à intégrer dans un composant unique l'ensemble des fonctions fluidiques, électriques et optiques.

Suivant d'autres modes de réalisation particuliers, le procédé et le dispositif associés peuvent être mis en oeuvre pour la mesure de particules autres que des cellules biologiques, telles que par exemple des particules céramiques dans le cadre de la fabrication de poudres industrielles, ou des pigments dans le cadre de la fabrication de peintures.

## Revendications

1. Procédé de cytométrie en flux sans fluides de gainage pour la caractérisation de particules, comprenant des étapes de :
- transfert d'un fluide (3) contenant des particules (4) au travers d'une cuve-orifice (2), laquelle cuve-orifice étant au moins en partie sensiblement transparente à au moins une longueur d'onde d'intérêt, et au moins en partie sensiblement isolante à l'électricité,
- mesure de variation d'impédance électrique générée par le passage desdites particules (4) contenues dans le fluide (3) au travers de la cuve-orifice (2),
- mesure d'au moins une propriété optique desdites particules (4), lesquelles interagissant avec au moins un faisceau de lumière (7) traversant l'orifice (1) de la cuve-orifice (2) dans une zone de mesure optique,
- caractérisation desdites particules (4) en exploitant lesdites mesures de variation d'impédance électrique et de propriétés optiques,
**caractérisé en ce qu'**il comprend en outre des étapes suivantes de:
- génération, au moyen d'une diode électroluminescente, d'un faisceau de taille supérieure au diamètre de l'orifice (1),
- déduction d'information sur la trajectoire des particules (4) dans la cuve-orifice (2), au moins à partir de l'analyse de ladite mesure de variation d'impédance électrique,
- utilisation de ladite information sur la trajectoire au moins pour l'exploitation desdites mesures de propriétés optiques lors de la caractérisation des particules en classant lesdites mesures en au moins deux catégories dites valides et non valides.

2. Procédé selon la revendication 1, **caractérisé en ce que** seul le fluide (3) contenant des particules (4) est transféré au travers de la cuve-orifice (2).

3. Procédé selon la revendication 1 ou 2, **caractérisé en ce que** la mesure de variation d'impédance électrique comprend une mesure de résistivité effectuée en courant continu et/ou au moins une mesure d'impédance complexe effectuée à au moins une fréquence non nulle.

4. Procédé selon l'une quelconque des revendications précédentes, **caractérisé en ce que** l'au moins une propriété optique mesurée est l'une des propriétés optiques suivantes : absorbance, diffusion élastique selon au moins un angle, diffusion inélastique selon au moins un angle, rétrodiffusion élastique, rétrodiffusion inélastique, autofluorescence, fluorescence à au moins une longueur d'onde provoquée par au moins un marqueur greffé sur les particules, fluorescence à au moins une longueur d'onde provoquée par au moins un marqueur greffé à au moins un élément contenu dans les particules, polarisation de la lumière.

5. Procédé selon l'une quelconque des revendications précédentes, **caractérisé en ce que** la caractérisation de particules comprend une classification des particules (4).

6. Procédé selon la revendication 5, **caractérisé en ce que** la caractérisation de particules comprend un dénombrement des particules (4).

7. Procédé selon la revendication 5 ou 6, **caractérisé en ce que** l'analyse de la variation d'impédance électrique comprend une comparaison entre la variation d'impédance électrique mesurée et au moins un élément de comparaison parmi au moins un modèle de variation d'impédance électrique et au moins une valeur numérique préalablement définie, le résultat de ladite comparaison déterminant si la trajectoire des particules (4) a traversé une zone de la cuve-orifice (2) préalablement définie.

8. Procédé selon la revendication 7, **caractérisé en ce que** les informations relatives aux particules (4) dont la trajectoire n'a pas traversé une zone de la cuve-orifice (2) préalablement définie sont retirées des données utilisées pour la classification desdites particules (4).

9. Procédé selon la revendication 7, **caractérisé en ce que**
- la propriété optique mesurée des particules (4) est comparée avec au moins un élément de comparaison parmi au moins un modèle de propriété optique et au moins une valeur numérique préalablement définie,
- si les résultats des comparaisons de la variation d'impédance électrique et de la propriété optique avec leurs modèles ou valeurs numériques préalablement définies respectifs ne satisfont pas au moins un critère préalablement défini, les informations relatives aux particules (4) sont retirées des données utilisées pour la classification desdites particules (4).

10. Procédé selon l'une quelconque des revendications 5 à 9, **caractérisé en ce que** l'opération de classification des particules (4) comprend des étapes de :
- attribution aux particules d'un ensemble de valeurs ou coordonnées issues de l'analyse des mesures effectuées lors de leur passage dans la cuve-orifice (2), lesquelles coordonnées définissant leur position dans un espace de représentation préalablement défini,
- segmentation dudit espace de représentation en régions distinctes, lesquelles régions regroupant des particules de caractéristiques sensiblement similaires.

11. Procédé selon l'une quelconque des revendications précédentes, **caractérisé en ce que** les particules (4) comprennent des cellules sanguines.

12. Dispositif de cytométrie en flux sans fluides de gainage pour la caractérisation de particules comprenant :
- une cuve-orifice (2) au moins en partie sensiblement transparente à au moins une longueur d'onde d'intérêt, et au moins en partie sensiblement isolante à l'électricité,
- des moyens de transfert d'un fluide (3) contenant des particules (4) au travers de ladite cuve-orifice,
- des moyens de mesure de variation d'impédance électrique générée par le passage desdites particules (4) contenues dans le fluide (3) au travers de la cuve-orifice (2),
- des moyens de mesure d'au moins une propriété optique desdites particules (4), lesquelles particules interagissant avec au moins un faisceau de lumière (7) traversant l'orifice (1) de la cuve-orifice (2) dans une zone de mesure optique,
- des moyens d'analyse desdites mesures de variation d'impédance électrique et de propriétés optiques,
**caractérisé en ce qu'**il comprend :
- une diode électroluminescente pour générer un faisceau de taille supérieure au diamètre de l'orifice (1),
- des moyens de déduction d'information sur la trajectoire des particules dans la cuve-orifice (2), au moins à partir de l'analyse de ladite mesure de variation d'impédance électrique, et
- des moyens d'utilisation de ladite information sur la trajectoire au moins pour l'exploitation desdites mesures de propriétés optiques lors de la caractérisation des particules en classant lesdites mesures en au moins deux catégories dites valides et non valides.

13. Dispositif selon la revendication 12, **caractérisé en ce que** la géométrie de la cuve-orifice (2) est adaptée de telle sorte que la variation d'impédance électrique mesurée lors du passage desdites particules (4) dépende de manière sensible de leur trajectoire dans ladite cuve-orifice (2).

14. Dispositif selon l'une des revendications 12 ou 13, **caractérisé en ce que** la cuve-orifice (2) comprend une section transversale interne sensiblement de l'une des formes suivantes : circulaire, rectangulaire.

15. Dispositif selon l'une quelconque des revendications 12 à 14, **caractérisé en ce que** la cuve-orifice (2) est constituée de l'un au moins des matériaux suivants : saphir, rubis, verre, plastique.

16. Dispositif selon l'une quelconque des revendications 12 à 15, **caractérisé en ce que** les moyens de mesure de variation d'impédance électrique générée par le passage des particules (4) comprennent au moins deux électrodes (5) disposées respectivement de part et d'autre de l'orifice (1) de la cuve-orifice (2), lesquelles électrodes étant en contact électrique avec le fluide (3) contenant les particules (4).

17. Dispositif selon la revendication 16, **caractérisé en ce que** les moyens de mesure de variation d'impédance électrique générée par le passage des particules (4) comprennent au moins une électrode disposée dans l'orifice (1) de la cuve-orifice (2), laquelle électrode étant en contact électrique avec le fluide (3) contenant les particules (4).

18. Dispositif selon l'une quelconque des revendications 12 à 17, **caractérisé en ce que** les moyens de mesure de la propriété optique des particules comprennent :
- au moins une source de lumière (8), et
- au moins un moyen de détection optoélectronique (13).

19. Dispositif selon la revendication 18, **caractérisé en ce que** les moyens de mesure de la propriété optique des particules comprennent au moins un moyen optique de focalisation (9) de la lumière (7) issue de la source de lumière (8) dans la cuve-orifice (2).

20. Dispositif selon la revendication 18 ou 19, **caractérisé en ce que** les moyens de mesure de la propriété optique des particules comprennent au moins un moyen optique de collecte (11) de la lumière (10) issue de la cuve-orifice (2).

21. Dispositif selon l'une quelconque des revendications 18 à 20, **caractérisé en ce que** au moins un des moyens de mesure de la propriété optique des particules est intégré à la cuve-orifice (2).

22. Appareil d'analyse sanguine **caractérisé en ce qu'**il comprend au moins un dispositif selon l'une quelconque des revendications 12 à 21, les particules (4) à analyser comprenant des cellules sanguines, et notamment des leucocytes.

## Patentansprüche

1. Verfahren zur Durchflusszytometrie ohne Hüllfluide zum Charakterisieren von Partikeln, umfassend die Schritte:
- Leiten eines Partikel (4) enthaltenden Fluids (3) durch eine Küvette (2), welche Küvette für zumindest eine interessierende Wellenlänge zumindest teilweise im Wesentlichen transparent ist und zumindest teilweise im Wesentlichen elektrisch isolierend ist,
- Messen der Änderung der elektrischen Impedanz, die durch den Durchgang der in dem Fluid (3) enthaltenen Partikel (4) durch die Küvette (2) erzeugt wird,
- Messen von zumindest einer optischen Eigenschaft der Partikel (4), die mit zumindest einem Lichtstrahl (7) in Wechselwirkung treten, der durch die Öffnung (1) der Küvette (2) in einen optischen Messbereich tritt,
- Charakterisieren der Partikel (4) durch Auswerten der Messungen der Änderung der elektrischen Impedanz und der optischen Eigenschaften, **dadurch gekennzeichnet, dass** es weiterhin die folgenden Schritte umfasst:
- Erzeugen eines Strahls mittels einer Leuchtdiode, dessen Abmessung Strahl größer als der Durchmesser der Öffnung (1) ist,
- Ableiten von Informationen über die Bewegungsbahn der Partikel (4) in der Küvette (2), zumindest ausgehend von der Analyse der Messung der Änderung der elektrischen Impedanz,
- Verwenden der Informationen über die Bewegungsbahn zumindest für die Auswertung der Messungen der optischen Eigenschaften bei der Charakterisierung der Partikel durch Einordnen der Messungen in zumindest zwei Kategorien, die als gültig und ungültig bezeichnet werden.

2. Verfahren nach Anspruch 1, **dadurch gekennzeichnet, dass** nur das Partikel (4) enthaltende Fluid (3) durch die Küvette (2) geleitet wird.

3. Verfahren nach Anspruch 1 oder 2, **dadurch gekennzeichnet, dass** die Messung der Änderung der elektrischen Impedanz eine Widerstandsmessung, die mit Gleichstrom erfolgt, und/oder zumindest eine Messung der komplexen Impedanz, die bei zumindest einer von Null verschiedenen Frequenz erfolgt, umfasst.

4. Verfahren nach einem der vorangehenden Ansprüche, **dadurch gekennzeichnet, dass** die zumindest eine gemessene optische Eigenschaft eine der nachfolgenden optischen Eigenschaften ist: Extinktion, elastische Streuung unter zumindest einem Winkel, unelastische Streuung unter zumindest einem Winkel, elastische Rückstreuung, unelastische Rückstreuung, Autofluoreszenz, Fluoreszenz bei zumindest einer Wellenlänge, hervorgerufen durch zumindest einen hinzugefügten Marker auf den Partikeln, Fluoreszenz bei zumindest einer Wellenlänge, hervorgerufen durch zumindest einen hinzugefügten Marker auf zumindest ein Element, das in den Partikeln enthalten ist, Polarisation des Lichts.

5. Verfahren nach einem der vorangehenden Ansprüche, **dadurch gekennzeichnet, dass** das Charakterisieren der Partikel ein Klassifizieren der Partikel (4) umfasst.

6. Verfahren nach Anspruch 5, **dadurch gekennzeichnet, dass** das Charakterisieren der Partikel ein Zählen der Partikel (4) umfasst.

7. Verfahren nach Anspruch 5 oder 6, **dadurch gekennzeichnet, dass** die Analyse der Änderung der elektrischen Impedanz einen Vergleich zwischen der gemessenen Änderung der elektrischen Impedanz und zumindest einem Vergleichselement aus zumindest einem Modell der Änderung der elektrischen Impedanz und zumindest einem zuvor definierten numerischen Wert umfasst, wobei das Ergebnis des Vergleichs bestimmt, ob die Bewegungsbahn der Partikel (4) einen zuvor definierten Bereich der Küvette (2) passiert hat.

8. Verfahren nach Anspruch 7, **dadurch gekennzeichnet, dass** die Informationen über die Partikel (4), deren Bewegungsbahnen keinen zuvor definierten Bereich der Küvette (2) passiert haben, aus den für das Klassifizieren der Partikel (4) verwendeten Daten entfernt werden.

9. Verfahren nach Anspruch 7, **dadurch gekennzeichnet, dass**
- die gemessene optische Eigenschaft der Partikel (4) mit zumindest einem Vergleichselement aus zumindest einem Modell der optischen Eigenschaft und zumindest einem zuvor definierten numerischen Wert verglichen wird,
- wenn die Ergebnisse der Vergleiche der Änderung der elektrischen Impedanz und der optischen Eigenschaft mit deren jeweiligen Modellen oder zuvor definierten numerischen Werten nicht zumindest einem zuvor definierten Kriterium entsprechen, die Informationen über die Partikel (4) von den für das Klassifizieren der Partikel (4) verwendeten Daten entfernt werden.

10. Verfahren nach einem der Ansprüche 5 bis 9, **dadurch gekennzeichnet, dass** der Vorgang des Klassifizierens der Partikel (4) die Schritte umfasst:
- Zuordnen von Werten oder Koordinaten aus der Analyse der bei deren Passieren durch die Küvette (2) erfolgten Messungen (zu den Partikeln eines Satzes), wobei diese Koordinaten ihre Position in einem zuvor definierten Repräsentationsraum definieren,
- Segmentieren des Repräsentationsraums in verschiedene Bereiche, wobei die Bereiche Partikel mit im Wesentlichen ähnlichen Eigenschaften zusammenfassen.

11. Verfahren nach einem der vorangehenden Ansprüche, **dadurch gekennzeichnet, dass** die Partikel (4) Blutzellen enthalten.

12. Vorrichtung zur Durchflusszytometrie ohne Hüllfluide zum Charakterisieren von Partikeln, enthaltend:
- eine Küvette (2), die für zumindest eine interessierende Wellenlänge zumindest teilweise im Wesentlichen transparent ist und zumindest teilweise im Wesentlichen elektrisch isolierend ist,
- Einrichtungen zum Leiten eines Partikel (4) enthaltenden Fluids (3) durch die Küvette,
- Einrichtungen zum Messen der Änderung der elektrischen Impedanz, die durch das Passieren der in dem Fluid (3) enthaltenen Partikel (4) durch die Küvette (2) erzeugt wird,
- Einrichtungen zum Messen von zumindest einer optischen Eigenschaft der Partikel (4), wobei die Partikel mit zumindest einem Lichtstrahl (7) in Wechselwirkung treten, der durch die Öffnung (1) der Küvette (2) in einen optischen Messbereich tritt,
- Einrichtungen zum Analysieren der Messungen der Änderung der elektrischen Impedanz und der optischen Eigenschaften,
**dadurch gekennzeichnet, dass** sie enthält:
- eine Leuchtdiode zum Erzeugen eines Strahls, dessen Abmessung größer als der Durchmesser der Öffnung (1) ist,
- Einrichtungen zum Ableiten von Informationen über die Bewegungsbahn der Partikel (4) in der Küvette (2), zumindest aus der Analyse der Messung der Änderung der elektrischen Impedanz; und
- Einrichtungen zum Verwenden der Informationen über die Bewegungsbahn zumindest für die Auswertung der Messungen der optischen Eigenschaften bei der Charakterisierung der Partikel durch Einordnen der Messungen in zumindest zwei Kategorien, die als gültig und ungültig bezeichnet werden.

13. Vorrichtung nach Anspruch 12, **dadurch gekennzeichnet, dass** die Geometrie der Küvette (2) so angepasst ist, dass die Änderung der elektrischen Impedanz, die beim Passieren der Partikel (4) gemessen wird, wesentlich von deren Bewegungsbahn in der Küvette (2) abhängt.

14. Vorrichtung nach einem der Ansprüche 12 oder 13, **dadurch gekennzeichnet, dass** die Küvette (2) einen Innenquerschnitt hat, der im Wesentlichen eine der folgenden Formen hat: kreisförmig, rechteckförmig.

15. Vorrichtung nach einem der Ansprüche 12 bis 14, **dadurch gekennzeichnet, dass** die Küvette (2) aus zumindest einem der nachfolgenden Materialien besteht: Saphir, Rubin, Glas, Kunststoff.

16. Vorrichtung nach einem der Ansprüche 12 bis 15, **dadurch gekennzeichnet, dass** die Einrichtungen zum Messen der Änderung der elektrischen Impedanz, die durch das Passieren der Partikel (4) hervorgerufen wird, zumindest zwei Elektroden (5) enthalten, die beiderseits der Öffnung (1) der Küvette (2) angeordnet sind, wobei die Elektroden elektrisch in Kontakt mit dem die Partikel (4) enthaltenden Fluid (3) sind.

17. Vorrichtung nach Anspruch 16, **dadurch gekennzeichnet, dass** die Einrichtungen zum Messen der Änderung der elektrischen Impedanz, die durch das Passieren der Partikel (4) hervorgerufen wird, zumindest eine Elektrode enthalten, die in der Öffnung (1) der Küvette (2) angeordnet ist, wobei die Elektrode elektrisch in Kontakt mit dem die Partikel (4) enthaltenden Fluid (3) ist.

18. Vorrichtung nach einem der Ansprüche 12 bis 17, **dadurch gekennzeichnet, dass** die Einrichtungen zum Messen der optischen Eigenschaft der Partikel enthalten:
- zumindest eine Lichtquelle (8), und
- zumindest eine optoelektronische Erfassungseinrichtung (13).

19. Vorrichtung nach Anspruch 18, **dadurch gekennzeichnet, dass** die Einrichtungen zum Messen der optischen Eigenschaft der Partikel zumindest eine optische Fokussierungseinrichtung (9) zum Fokussieren des von der Lichtquelle (9) stammenden Lichts (7) in der Küvette (2) enthalten.

20. Vorrichtung nach Anspruch 18 oder 19, **dadurch gekennzeichnet, dass** die Einrichtungen zum Messen der optischen Eigenschaft der Partikel zumindest eine optische Sammeleinrichtung (11) zum Sammeln des von der Küvette (2) stammenden Lichts (10) enthalten.

21. Vorrichtung nach einem der Ansprüche 18 bis 20, **dadurch gekennzeichnet, dass** zumindest eine der Einrichtungen zum Messen der optischen Eigenschaft der Partikel in der Küvette (2) integriert sind.

22. Blutanalysegerät, **dadurch gekennzeichnet, dass** es zumindest eine Vorrichtung nach einem der Ansprüche 12 bis 21 enthält, wobei die zu analysierenden Partikel (4) Blutzellen, insbesondere Leukozyten, enthalten.

## Claims

1. Method of flow cytometry without sheath fluids for particle characterization, comprising steps of:
- transfer of a fluid (3) containing particles (4) through an aperture flow-cell (2), said aperture flow-cell being at least in part substantially transparent to at least one wavelength of interest, and at least in part substantially insulating to electricity,
- measurement of electrical impedance variation generated by the passage of said particles (4) contained in the fluid (3) through the aperture flow-cell (2),
- measurement of at least one optical property of said particles (4), which interact with at least one light beam (7) passing through the orifice (1) of the flow-cell (2) in an optical measurement zone,
- characterization of said particles (4) by using said measurements of electrical impedance variation and optical properties,
**characterized in that** it further comprises following steps of:
- generating, by means of an electroluminescent diode, a beam having a size larger than the diameter of the orifice (1),
- deducing information on the trajectory of the particles (4) in the aperture flow-cell (2), at least from the analysis of said measurement of electrical impedance variation,
- use of said information on the trajectory at least for the exploitation of said measurements of optical properties during characterization of the particles by classifying said measurements in at least two categories, called valid and invalid.

2. Method according to claim 1, **characterized in that** only the fluid (3) containing particles (4) is passed through the aperture flow-cell (2).

3. Method according to claim 1 or 2, **characterized in that** the measurement of the electrical impedance variation comprises a measurement of resistivity carried out with direct current and/or at least one complex impedance measurement carried out at at least one non-zero frequency.

4. Method according to any one of the preceding claims, **characterized in that** the at least one optical property measured is one of the following optical properties: absorbance, elastic scattering at at least one angle, inelastic scattering at at least one angle, elastic backscattering,
inelastic backscattering, autofluorescence, fluorescence at at least one wavelength caused by at least one marker attached to the particles, fluorescence at at least one wavelength caused by at least one marker attached to at least one element contained in the particles, polarization of light.

5. Method according to any one of the preceding claims, **characterized in that** the particle characterization comprises a classification of the particles (4).

6. Method according to claim 5, **characterized in that** the particle characterization comprises a counting of the particles (4).

7. Method according to claim 5 or 6, **characterized in that** the analysis of the electrical impedance variation comprises a comparison between the measured electrical impedance variation and at least one element of comparison among at least one model of electrical impedance variation and at least one previously defined numerical value, the result of said comparison determining whether the trajectory of the particles (4) has traversed a previously defined zone of the aperture flow-cell (2).

8. Method according to claim 7, **characterized in that** the data relating to the particles (4) whose trajectory did not pass through a previously defined zone of the aperture flow-cell (2) is removed from the data used for the classification of said particles (4).

9. Method according to claim 7, **characterized in that**
- the measured optical property of the particles (4) is compared with at least one element of comparison among at least one model of optical property and at least one previously defined numerical value,
- if the results of the comparisons of the electrical impedance variation and of the optical property with their respective models or previously defined numerical values do not satisfy at least one previously defined criterion, the data relating to the particles (4) is removed from the data used for the classification of said particles (4).

10. Method according to any one of claims 5 to 9, **characterized in that** the operation of classification of the particles (4) comprises steps of:
- attribution to the particles of a set of values or coordinates obtained from the analysis of the measurements carried out during their passage through the aperture flow-cell (2), said coordinates defining their position in a previously defined representation space,
- segmentation of said representation space into distinct regions, said regions grouping together particles of substantially similar characteristics.

11. Method according to any one of the preceding claims, **characterized in that** the particles (4) comprise blood cells.

12. Flow cytometry device without sheath fluids for particle characterization comprising:
- an aperture flow-cell (2) at least in part substantially transparent to at least one wavelength of interest, and at least in part substantially insulating to electricity,
- means for transfer of a fluid (3) containing particles (4) through said aperture flow-cell,
- means for measurement of the electrical impedance variation generated by the passage of said particles (4) contained in the fluid (3) through the aperture flow-cell (2),
- means for measurement of at least one optical property of said particles (4), said particles interacting with at least one light beam (7) passing through the orifice (1) of the flow-cell (2) in an optical measurement zone,
- means for analysis of said measurements of variation in electrical impedance and optical properties,
**characterized in that** it comprises:
- an electroluminescent diode for generating a beam having a size larger than the diameter of the orifice (1),
- means for deducing information on the trajectory of the particles in the aperture flow-cell (2), at least from the analysis of said measurement of the electrical impedance variation, and
- means for using said information on the trajectory at least for the exploitation of said measurements of optical properties during characterization of the particles by classifying said measurements in at least two categories, called valid and invalid.

13. Device according to claim 12, **characterized in that** the geometry of the aperture flow-cell (2) is adapted in such a way that the measured electrical impedance variation during passage of said particles (4) depends substantially on their trajectory in said aperture flow-cell (2).

14. Device according to one of claims 12 or 13, **characterized in that** the aperture flow-cell (2) comprises an internal cross-section substantially of one of the following forms: circular, rectangular.

15. Device according to any one of claims 12 to 14, **characterized in that** the aperture flow-cell (2) is constituted of at least one of the following materials: sapphire, ruby, glass, plastic.

16. Device according to any one of claims 12 to 15, **characterized in that** the means for measurement of the electrical impedance variation generated by the passage of the particles (4) comprise at least two electrodes (5) arranged respectively on either side of the orifice (1) of the aperture flow-cell (2), said electrodes being in electrical contact with the fluid (3) containing the particles (4).

17. Device according to claim 16, **characterized in that** the means for measurement of the electrical impedance variation generated by the passage of the particles (4) comprise at least one electrode arranged in the orifice (1) of the aperture flow-cell (2), said electrode being in electrical contact with the fluid (3) containing the particles (4).

18. Device according to any one of claims 12 to 17, **characterized in that** the means for measurement of the optical property of the particles comprise:
- at least one light source (8), and
- at least one optoelectronic detecting means (13).

19. Device according to claim 18, **characterized in that** the means for measurement of the optical property of the particles comprise at least one optical means of focusing (9) of the light (7) emitted from the light source (8) in the aperture flow-cell (2).

20. Device according to claim 18 or 19, **characterized in that** the means for measurement of the optical property of the particles comprise at least one optical means of collecting (11) of the light (10) emitted from the aperture flow-cell (2).

21. Device according to any one of claims 18 to 20, **characterized in that** at least one of the means for measurement of the optical property of the particles is integrated with the aperture flow-cell (2).

22. Instrument for blood analysis, **characterized in that** it comprises at least one device according to any one of claims 12 to 21, the particles (4) to be analysed comprising blood cells, and notably leukocytes.
